# EUROPEAN PATENT APPLICATION

(11) **EP 4 592 243 A1**
(43) Date of publication of application: **30.07.2025**
(21) Application number: 23868162.1
(22) Date of filing: 19.09.2023
(51) Int. Cl.: C01B 13/02, A61M 16/10, B01D 53/053

(54) **OXYGEN CONCENTRATION DEVICE**

(30) Priority: 22.09.2022 JP 2022150827
(71) Applicant: Teijin Pharma Limited, Tokyo 100-0013 (JP)
(72) Inventor: MORISHITA, Yuta, Tokyo 100-0013 (JP); MUROSHIGE, Yoshihiro, Tokyo 100-0013 (JP)
(74) Representative: Hoffmann Eitle
(86) International application number: PCT/JP2023/033854
(87) International publication number: WO 2024/063041

(57) **Abstract**

[Problem]

To detect an abnormality in a flow path switching valve upstream of an oxygen concentrator from the waveform of a pressure sensor provided in a product tank.

[Solution]

The oxygen concentrator is provided with a monitoring means for monitoring conditions of the flow path switching means based on the output of the pressure sensor in the product tank, and detects an abnormality of the flow path switching valve when a detected value of the pressure sensor during the adsorption step and/or desorption step falls outside a predetermined threshold range.

## Description

### [Technical Field]

The present invention relates to a pressure swing adsorption-type oxygen concentrator that separates oxygen from air.

### [Background Art]

Respiratory diseases such as asthma, pulmonary emphysema, and chronic bronchitis, and viral pneumonia recently caused by COVID-19 are afflicting increasing number of patients, and one of the most effective therapies for them is oxygen inhalation therapy. Such oxygen inhalation therapy is a therapy in which a patient suffering from a respiratory disease inhales oxygen gas or oxygen-enriched gas. Sources of oxygen for this therapy include those known sources such as an oxygen concentrator, liquid oxygen, and an oxygen gas cylinder, and in home oxygen therapy, oxygen concentrators are mainly used due to the simpleness of use and easiness of maintenance.

An oxygen concentrator is a device that separates and concentrates the approximately 21% of oxygen present in the air and supplies concentrated oxygen. Known examples of such devices include a membrane oxygen concentrator that uses a membrane selectively allowing oxygen to permeate, and a pressure swing adsorption-type oxygen concentrator that uses an adsorbent selectively adsorbing nitrogen or oxygen, and a pressure swing adsorption-type oxygen concentrator is mainly used in home oxygen therapy due to production of oxygen at a high concentration of 90% or more.

A pressure swing adsorption-type oxygen concentrator can continuously produce high concentration oxygen-enriched gas by alternately repeating a pressurization/adsorption step and a decompression/desorption step in an adsorption cylinder filled with molecular sieve zeolite, an adsorbent selectively adsorbing nitrogen over oxygen, such as 5A type, 13X type, or Li-X type; in the former step, air compressed by a compressor is supplied to the cylinder, bringing nitrogen to be adsorbed on the adsorbent under pressurized conditions to obtain unadsorbed oxygen, and in the latter step, the pressure in the cylinder is reduced to atmospheric pressure or below, bringing the nitrogen adsorbed on the adsorbent to be purged to regenerate the adsorbent.

### [Citation List]

### [Patent Literature]

[PTL 1] JP-A-2018-175542

### [Summary of Invention]

### [Technical Problem]

An oxygen concentrator using a compressor as a pressurized air source is equipped with a pressure sensor on the compressor discharge side for detection of the adsorption pressure, and is also equipped with a safety mechanism such as detection of pressure abnormalities caused by compressor malfunctions and sounding an alarm, and reduction of the compressor speed or stop of the compressor. There is also equipped with a relief valve to ensure the safety of the equipment.

Further, as described in PTL 1, an oxygen concentrator is also known to have a function for detecting an abnormality in an intake valve or an exhaust valve based on the detection result of a compressor pressure.

Since the feed air contains a large amount of water vapor, the downstream side of the compressor becomes a high-humidity environment under pressurized conditions. This causes a problem of forming condensation inside the tube connecting the pressure sensor, which requires a solution of dehumidifying the feed air or using an expensive anti-condensation tube for the connecting tube. In contrast, the downstream side of the adsorption cylinder of the oxygen concentrator is dehumidified by the adsorbent and is in a dry oxygen environment, and thus detecting the pressure inside the product tank does not require such dehumidification equipment.

However, the pressure in the product tank varies depending on the oxygen generation conditions and the timing in the adsorption/desorption step, and thus variation of the product tank pressure hardly facilitates locating a malfunctioning one among the components possibly causing the pressure abnormality in the upstream of the product tank, such as compressor, adsorption cylinder, and flow path switching valve.

Further, since the oxygen concentrator adjusts the amount of oxygen produced in accordance with the amount of oxygen to be supplied, the adsorption pressure and product tank pressure vary greatly with the control of the compressor speed even under normal conditions.

### [Solution to Problem]

The present inventors have found a method for locating an abnormality in the upstream flow path switching valve from the waveform of a pressure sensor provided in the product tank.

The present invention provides the following oxygen concentrator.
1. An oxygen concentrator comprising: a plurality of adsorption cylinders filled with an adsorbent selectively adsorbing nitrogen over oxygen;
   a compressor for supplying pressurized air to the adsorption cylinder;
   a flow path switching valve equipped with a supply valve for supplying pressurized air to each of the adsorption cylinders and an exhaust valve for discharging exhaust gas from the adsorption cylinders to outside the system;
   a product tank for temporarily storing the oxygen generated from the adsorption cylinder; and
   a pressure sensor for detecting a product tank pressure;
      the oxygen concentrator being a pressure swing adsorption-type oxygen concentrator continuously producing oxygen by sequentially switching and repeating an adsorption step of producing oxygen under pressurized conditions and a desorption step of exhausting nitrogen under reduced pressure in each adsorption cylinder,
   wherein the oxygen concentrator is provided with a monitoring means for monitoring conditions of the flow path switching means based on the output of the pressure sensor, and detects an abnormality of the flow path switching valve when a detected value of the pressure sensor during the adsorption step and/or desorption step falls outside a predetermined threshold range, i.e., falls outside a predetermined range on a positive or negative side.
2. The oxygen concentrator according to clause 1, wherein the monitoring means determines that a flow path switching valve is abnormal when a difference/ratio is larger than a predetermined threshold value from/to product tank pressures at a specific timing during the adsorption step or desorption step of a plurality of adsorption cylinders.
3. The oxygen concentrator according to clause 2, wherein the specific timing is a predetermined time until the end of an adsorption step or a desorption step.
4. The oxygen concentrator according to clause 1, wherein regarding one of a plurality of adsorption cylinders, when a product tank pressure at the end of an adsorption step is larger than a product tank pressure at the start of the adsorption step by more than a predetermined threshold value, and a product tank pressure at the end of a desorption step is larger than a product tank pressure at the start of the desorption step by more than a predetermined threshold value, the monitoring means determines that abnormal is a supply valve supplying pressurized air to the adsorption cylinder.
5. The oxygen concentrator according to clause 1, wherein regarding one of a plurality of adsorption cylinders, when a product tank pressure at the end of a desorption step is smaller than a product tank pressure at the start of an adsorption step by less than a predetermined threshold value, and a product tank pressure at the end of an adsorption step is larger than a product tank pressure at the start of the adsorption step by more than a predetermined threshold value, the monitoring means determines that abnormal is an exhaust valve releasing exhaust gas from the adsorption cylinder to outside the system.
6. The oxygen concentrator according to clause 1, wherein regarding an adsorption step or desorption step of each adsorption cylinder, when a difference/ratio of the maximum pressure from/to the minimum pressure of a product tank is larger than a predetermined threshold value, the monitoring means determines that a flow path switching valve is abnormal.
7. The oxygen concentrator according to clause 1, wherein regarding each adsorption cylinder, when a difference/ratio of the maximum pressure of a product tank during an adsorption step from/to that during a desorption step is larger than a predetermined threshold value, the monitoring means determines that a flow path switching valve is abnormal.
8. The oxygen concentrator according to any one of clauses 1 to 7, wherein the predetermined threshold value is a value determined based on a product tank pressure set in accordance with oxygen supply conditions of the oxygen concentrator.
9. The oxygen concentrator according to clause 8, wherein the oxygen supply conditions are a set flow rate, an oxygen supply mode of continuous flow or respiration synchronized flow, an adsorption cylinder pressure, or a compressor rotation speed for the oxygen concentrator.

### [Advantageous Effects of Invention]

The oxygen concentrator of the present invention allows the pressure sensor for detecting the adsorption pressure to be provided in the product tank for storing dry oxygen-enriched gas, rather than provided on the compressor discharge side containing much water vapor. This allows detection of abnormalities in devices upstream of the product tank and abnormalities in flow path switching valves such as a supply valve and an exhaust valve from the relationship between change in the pressure waveform of the product tank and the switching timing of the adsorption/desorption step.

### [Brief Description of Drawings]

Fig. 1 shows a block diagram of an oxygen concentrator of the present invention.
Fig. 2 shows the change of product tank pressure in the oxygen concentrator device of the present invention.
Fig. 3 shows the change in product tank pressure when a supply valve is abnormal in the oxygen concentrator of the present invention.
Fig. 4 shows the change in product tank pressure when an exhaust valve is abnormal in the oxygen concentrator of the present invention.

### [Description of Embodiments]

Embodiments of the oxygen concentrator of the present invention will be described with reference to the drawings.

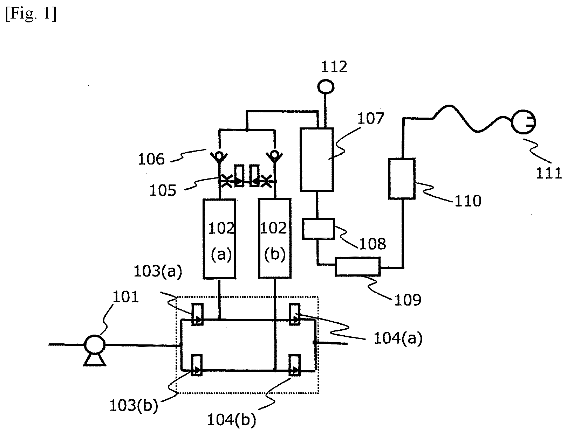

Fig. 1 is a schematic diagram illustrating the configuration of a pressure swing adsorption-type oxygen concentrator in one embodiment of the present invention. A pressure swing adsorption-type oxygen concentrator of the present invention is equipped with compressor 101 for supplying feed air, adsorption cylinder 102 filled with an adsorbent selectively adsorbing nitrogen over oxygen, and a flow path switching means for switching adsorption/desorption step of supply valve 103, exhaust valve 104, and pressure equalizing valve 105, and adjusts the flow rate of the oxygen-enriched gas separated and generated from the feed air to a predetermined value by flow rate setting devices such as pressure adjusting valve 108 and control valve 109, and after humidification by water humidifier 110, supplies the gas to the user via cannula 111.

Air normally contains approximately 21% oxygen gas, approximately 77% nitrogen gas, 0.8% argon gas, and 1.2% carbon dioxide etc. Such oxygen concentrator separates out oxygen required as breathing gas. The oxygen-enriched gas is separated in the adsorption step as follows: feed air is supplied from compressor 101 as pressurized air sequentially to adsorption cylinder 102 of feed air supply target, filled with an adsorbent such as zeolite selectively adsorbing nitrogen molecules over oxygen molecules, via a switched flow path by controlling opening/closing of supply valve 103 and exhaust valve 104, and in the pressurized adsorption cylinder, approximately 77% nitrogen gas in the feed air is selectively adsorbed/removed.

The adsorption cylinder consists of a cylindrical vessel filled with an adsorbent selectively adsorbing nitrogen over oxygen. The number of adsorption cylinders is determined in relation to the amount of oxygen produced, and a two-cylinder type shown in Fig. 1 or multi-cylinder type adsorption cylinder is preferably used for continuous and efficient production of oxygen-enriched gas from feed air.

The oxygen-enriched gas mainly composed of oxygen unadsorbed in the adsorption cylinder flows into product tank 107 via check valve 106 provided for prevention of backflow into the adsorption cylinder, and is temporarily stored there.

Continuous production of oxygen-enriched gas requires the nitrogen adsorbed to be desorbed/removed from the adsorbent packed in the adsorption cylinder. In the desorption step, the adsorption cylinder is connected to the exhaust line by closing the supply valve and opening the exhaust valve and is switched from a pressurized state to an atmospheric open state, and the adsorbent is regenerated by desorbing the nitrogen adsorbed under pressurization.

Two adsorption cylinders operate switching each step as staggered with each other to produce oxygen continuously: while one adsorption cylinder is at an adsorption step for producing oxygen, the other adsorption cylinder is at a desorption step for regenerating the adsorbent.

The following two steps are repeated alternately to continuously produce unadsorbed oxygen: an adsorption step of supplying pressurized air from compressor 101 via supply valve 103(a) to adsorption cylinder 102(a) and producing oxygen by adsorbing nitrogen; and a desorption step of depressurizing the other adsorption cylinder 102(b) and desorbing the adsorbed nitrogen to be exhausted to outside the system via exhaust valve 104(b). Meanwhile, the adsorption and desorption steps are repeated to produce oxygen-enriched gas while incorporating steps such as a pressure equalization step of equalizing the pressure of the adsorption cylinders via a pressure equalization valve, and a purging step of pressurizing the adsorption cylinder to generate oxygen while flowing a part of the oxygen to the other adsorption cylinder to increase the regeneration efficiency.

Oxygen-enriched gas is produced from feed air and temporarily stored in a product tank. The oxygen-enriched gas stored in this product tank contains high concentration oxygen gas of, for example, 95%, is supplied to a humidifier after controlling the supply flow rate and pressure by flow rate setting devices such as a pressure adjusting valve and a control valve, and the humidified oxygen-enriched gas is then supplied to the patient. Such a humidifier may be a bubbling type humidifier or a surface evaporation type humidifier that uses water as a humidification source.

The flow rate and oxygen concentration of the oxygen-enriched gas supplied to the user are detected by an ultrasonic oxygen concentration/flow rate sensor, which enables control of oxygen generation using feedback-control of the compressor rotation speed and the opening/closing time of the flow path switching valve based on the detected oxygen concentration value and the oxygen supply flow rate value.

The adsorbent selectively adsorbing nitrogen over oxygen to be filled in the adsorption cylinder, molecular sieve zeolites such as Na-X type, Li-X type, and MD-X type, adsorbs nitrogen and, at the same time, also adsorbs moisture in the air, which enables the oxygen-enriched gas produced to be separated as a nearly bone-dry gas. Continuous inhalation of such oxygen-enriched gas may cause drying of the nostrils etc., and for the purpose of preventing this, a water humidifier humidifying the oxygen-enriched gas by bubbling humidifying water or a waterless humidifier using a hollow fiber membrane that selectively allows water vapor in the air to pass through can be installed as necessary in the piping that connects the product tank temporarily storing the generated oxygen-enriched gas to the cannula supplying oxygen to the patient.

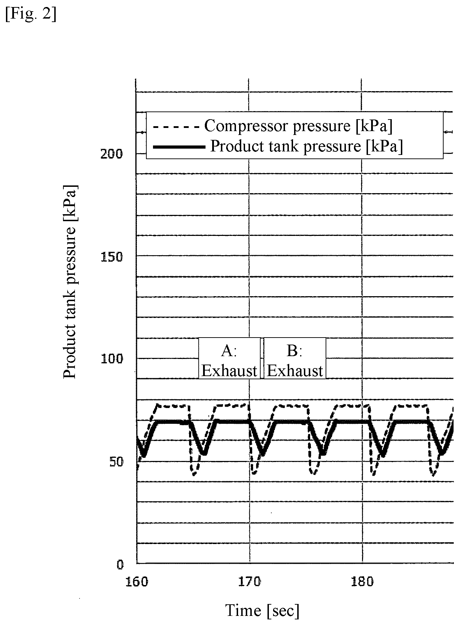

Fig. 2 shows a pressure waveform of pressure sensor 112 installed in product tank 107. The compressor pressure, which is the adsorption pressure, and the sequence of the adsorption/desorption step are controlled so as to maintain the constant amounts of oxygen production and oxygen supply to achieve energy conservation. A slight pressure drop was observed in the product tank during the pressure equalization step when switching between the adsorption and desorption steps. Although the steady-state pressure varies depending on the supply flow rate, the product tank pressure is maintained at a substantially constant value.

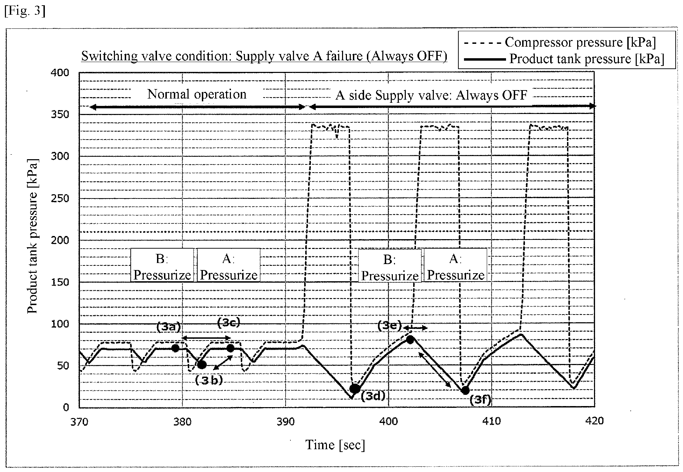

Fig. 3 shows the pressure waveform in the product tank when an abnormality occurs in the supply valves and one of the supply valves cannot be opened. The solenoid valves used for the supply valve, exhaust valve, etc. are designed to be open when the power is on and closed when the power is off so as to prevent deterioration of the adsorbent due to moisture absorption by closing the supply path to the adsorption cylinder when the system is stopped, and an abnormality in which the solenoid valve does not open even when voltage is applied accounts for a large portion of the abnormalities, but the opposite case may occur.

When one of the supply valves cannot be opened, the adsorption step cannot produce oxygen, and the product tank pressure will drop. When the step is switched to the desorption step, the supply valve supplying pressurized air to the other adsorption cylinder operates without abnormality, and oxygen is generated to restore the product tank pressure. Detecting such adsorption/desorption step and change of the product tank pressure enables detection of an abnormality in the supply valve.

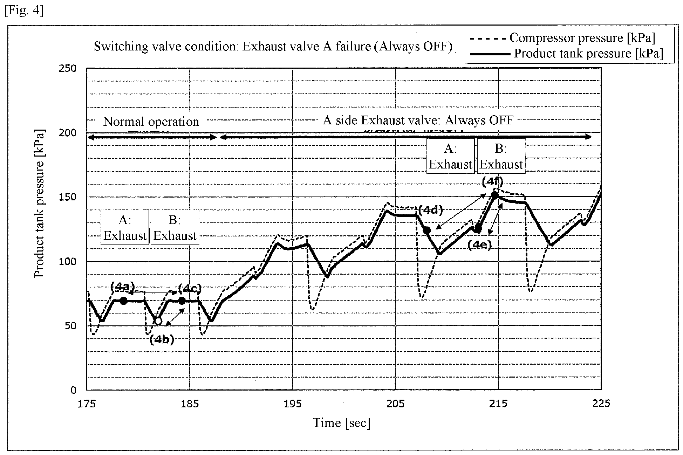

Fig. 4 shows the pressure waveform in the product tank when an abnormality occurs in the exhaust valves and one of the exhaust valves remains closed. When an exhaust valve fails and remains closed, the desorption step of the corresponding adsorption cylinder cannot exhaust desorbed nitrogen and purge gas. The adsorption step in the opposite adsorption cylinder also produces additional amount of oxygen gas for purge/exhaust, which increases the product tank pressure gradually. Even after switching of adsorption/desorption step, the adsorption cylinder on the adsorption step with the exhaust valve abnormality maintains high pressure, then is supplied with pressurized air to further increase the product tank pressure, and with the start of the purge/exhaust step, the amount of generated gas and the amount of supplied gas are balanced to give a constant product tank pressure. In contrast, in an adsorption cylinder with a normal exhaust valve, the adsorption cylinder pressure drops significantly at the start of the adsorption step due to pressure equalization between the adsorption cylinders and also the product tank pressure drops accordingly, and thereafter, repeats a pressure pattern of increase, constant pressure retention, and drop in accordance with the progress of adsorption/desorption step.

Therefore, the level of the product tank pressure alone cannot identify an abnormality in the flow path switching means such as the supply valve and exhaust valve.

The pressure swing adsorption-type oxygen concentrator of the present invention is equipped with a monitoring means for monitoring the condition of the flow path switching means based on the output of the pressure sensor in the product tank, and by simultaneously monitoring the product tank pressure and the stage of the adsorption step and/or desorption step involving switching of the flow path switching means, detects an abnormality in the flow path switching valve when the detected value of the pressure sensor is larger than a predetermined threshold value, i.e., falls outside a predetermined range on the positive or negative side.

Such a predetermined threshold value is a value determined based on the product tank pressure set in accordance with the oxygen supply conditions of the oxygen concentrator, such as a set flow rate, a supply mode such as continuous supply or respiration synchronized supply, and a compressor rotation speed associated therewith.

The monitoring means determines that a flow path switching valve is abnormal when product tank pressures during an adsorption step of a plurality of adsorption cylinders, i.e., a product tank pressure at a specific timing during an adsorption step of adsorption cylinder (a) and a product tank pressure at the same timing during the subsequent adsorption step of adsorption cylinder (b), though normally showing the same pressure value (3a, 3c in Fig. 3), have a difference or ratio between both these two values larger than a predetermined threshold value. Such a specific timing is preferably set to a predetermined time until the end of the adsorption or desorption step, such as 10 seconds or 5 seconds until the end, during which the product tank pressure value stabilizes.

In addition, the monitoring means can determine that the flow path switching valve is abnormal, regarding an adsorption step or desorption step of each adsorption cylinder, when a difference of the maximum pressure from the minimum pressure or a ratio of both in the product tank during the step is larger than a predetermined threshold value as given by the pressure difference etc. under normal conditions, or regarding each adsorption cylinder, when a difference of the maximum pressures in the product tank during an adsorption step from that during a desorption step or a ratio thereof is larger than a predetermined threshold value.

Such a method, though capable of determining that the flow path switching valve is abnormal, cannot locate the abnormality.

The oxygen concentrator of the present invention is equipped with the monitoring means for the flow path switching means to monitor the sequence of the adsorption/desorption step and the product tank pressure, and as shown by the product tank pressure waveform in Fig. 3, when the product tank pressure (3f) at the end of the adsorption step of adsorption cylinder 102(a) is larger than the product tank pressure (3e) at the start of the desorption step by more than a predetermined threshold value (pressure difference between 3b and 3c), and the product tank pressure (3e) at the end of the desorption step is larger than the product tank pressure (3d) at the start of the desorption step by more than a predetermined threshold value, the monitoring means can determine that abnormal is supply valve 103(a) for supplying pressurized air to adsorption cylinder 102(a).

Further, as shown by the product tank pressure waveform in Fig. 4, when the product tank pressure (4e) at the end of the desorption step of adsorption cylinder 102(a) falls within a predetermined threshold value with respect to the product tank pressure (4d) at the start of the desorption step, and the product tank pressure (4f) at the end of the adsorption step is larger than the product tank pressure (4e) at the start of the adsorption step by more than a predetermined threshold value, the monitoring means can determine that abnormal is the exhaust valve 104(a) for releasing exhaust from the adsorption cylinder to outside the system.

### [Industrial Applicability]

The oxygen concentrator of the present invention can be applied to home oxygen inhalation therapy in which oxygen is supplied to patients with chronic respiratory diseases and the like as a medical device ensuring safety for the use.

### [Reference Signs List]

101 Compressor
102 Adsorption cylinder
103 Supply valve
104 Exhaust valve
105 Pressure equalizing valve
106 Check valve
107 Product tank
108 Pressure Regulating valve
109 Control valve
110 Water humidifier
111 Cannula
112 Pressure sensor

## Claims

1. An oxygen concentrator comprising: a plurality of adsorption cylinders filled with an adsorbent selectively adsorbing nitrogen over oxygen;
a compressor for supplying pressurized air to the adsorption cylinder;
a flow path switching valve equipped with a supply valve for supplying pressurized air to each of the adsorption cylinders and an exhaust valve for discharging exhaust gas from the adsorption cylinders to outside the system;
a product tank for temporarily storing the oxygen generated from the adsorption cylinder; and
a pressure sensor for detecting a product tank pressure;
the oxygen concentrator being a pressure swing adsorption-type oxygen concentrator continuously producing oxygen by sequentially switching and repeating an adsorption step of producing oxygen under pressurized conditions and a desorption step of exhausting nitrogen under reduced pressure in each adsorption cylinder,
wherein the oxygen concentrator is provided with a monitoring means for monitoring conditions of the flow path switching means based on the output of the pressure sensor, and detects an abnormality of the flow path switching valve when a detected value of the pressure sensor during the adsorption step and/or desorption step falls outside a predetermined threshold range.

2. The oxygen concentrator according to claim 1, wherein the monitoring means determines that a flow path switching valve is abnormal when a difference/ratio is larger than a predetermined threshold value from/to product tank pressures at a specific timing during the adsorption step or desorption step of a plurality of adsorption cylinders.

3. The oxygen concentrator according to claim 2, wherein the specific timing is a predetermined time until the end of an adsorption step or a desorption step.

4. The oxygen concentrator according to claim 1, wherein regarding one of a plurality of adsorption cylinders, when a product tank pressure at the end of an adsorption step is smaller than a product tank pressure at the start of the adsorption step by less than a predetermined threshold value, and a product tank pressure at the end of a desorption step is larger than a product tank pressure at the start of the desorption step by more than a predetermined threshold value, the monitoring means determines that abnormal is a supply valve supplying pressurized air to the adsorption cylinder.

5. The oxygen concentrator according to claim 1, wherein regarding one of a plurality of adsorption cylinders, when a product tank pressure at the end of a desorption step is larger than a product tank pressure at the start of an desorption step by more than a predetermined threshold value, and a product tank pressure at the end of an adsorption step is larger than a product tank pressure at the start of the adsorption step by more than a predetermined threshold value, the monitoring means determines that abnormal is an exhaust valve releasing exhaust gas from the adsorption cylinder to outside the system.

6. The oxygen concentrator according to claim 1, wherein regarding an adsorption step or desorption step of a plurality of adsorption cylinders, when a difference/ratio of the maximum pressure from/to the minimum pressure of a product tank is larger than a predetermined threshold value, the monitoring means determines that a flow path switching valve is abnormal.

7. The oxygen concentrator according to claim 1, wherein regarding a plurality of adsorption cylinders, when a difference/ratio of the maximum pressure of a product tank during an adsorption step from/to that during a desorption step is larger than a predetermined threshold value, the monitoring means determines that a flow path switching valve is abnormal.

8. The oxygen concentrator according to any one of claims 1 to 7, wherein the predetermined threshold value is a value determined based on a product tank pressure set in accordance with oxygen supply conditions of the oxygen concentrator.

9. The oxygen concentrator according to claim 8, wherein the oxygen supply conditions are a set flow rate, an oxygen supply mode of continuous flow or respiration synchronized flow, an adsorption cylinder pressure, or a compressor rotation speed for the oxygen concentrator.
